# EUROPEAN PATENT APPLICATION

(11) **EP 1 156 113 A1**
(43) Date of publication of application: **21.11.2001**
(21) Application number: 00110022.1
(22) Date of filing: 12.05.2000
(51) Int. Cl.: C12N 15/56, C12N 9/28, C12N 1/21

(54) **Corynebacterium glutamicum expressing heterologous amylase**

(71) Applicant: Degussa AG, 40474 Düsseldorf (DE)
(72) Inventor: Berens, Stephan, Dr., 33615 Bielefeld (DE); Kalinowski, Jörn, Dr., 33615 Bielefeld (DE); Pühler, Alfred, Prof.Dr., 33739 Bielefeld (DE)

(57) **Abstract**

The present invention refers to a Corynebacterium glutamicum strain which is able to use starch as a sole carbon source, since the strain contains a heterologous amylase gene, and the use of such a bacterial strain for production and secretion of desired product.

## Description

The present invention refers to a Corynebacterium glutamicum strain, which is able to use starch as a sole carbon source, since the strain contains a heterologous amylase gene, and the use of such a bacterial strain for production of desired products.

In the gram-positive, non sporulating soil bacterium Corynebacterium glutamicum, which is of special interest for the industrial production of fine chemicals (Wohlleben, W., Muth, G. and Kalinowski, J. (1993), Genetic Engineering of Microorganisms, pp. 83-133, edited by A. Pühler, New York: Weinheim), cell wall proteins are the major secretion products (Joliff, G., Mathieu, L., Hahn, V., Bayan, N., Duchiron, F., Renaud, M., Chechter, E. and Leblon, G. (1992), Mol. Microbiol. 6; 2349-2362). Due to this lack of extracellular protease activity and its simultaneous ability to secrete large amounts of proteins, Corynebacterium glutamicum is an ideal host for the production of heterologous exoproteins, e. g., as shown for the production of cellulase from Cellulomonas fimi (Paradis, F.W., Warren, R.A.J., Kilburn, D.G. and Miller Jr., R.C. (1987), Gene 61, 199-206) and ovine gamma interferon (Billman-Jacobe, H., Hodgson, A.I.M., Lightowlers, M., Wood, P.R. and Radford, A.J. (1994), Appl. Env. Microbiol 60, 1641-1645).

Corynebacterium glutamicum wild type uses a large variety of monomeric and oligomeric sugars like glucose, sucrose or maltose as energy source (Vahjen, W., Munch, J.-C. & Tebbe, C. C. (1995), FEMS Microb. Ecol. 18: 317-328)

Starch normally cannot be used as an energy source by this bacterium, since the wild type has no enzymes which can decompose starch, obtaining utilizable sugar.

Starch, however, is an unexpensive, easily obtainable, high energy source, which is soluble in water and therefore easily usable in media for bacterial fermentation.

Another bacterial strain, Steptomyces griseus contains a gene encoding α-amylase (amy gene), enabling this strain to use starch as energy source.

Object of the present invention is to provide a means for easy, unexpensive and effective production of fine chemicals, peptides and proteins. This object is met by a genetically modified Corynebacterium glutamicum strain which is able to use starch as a sole carbon source.

The usage of starch has a great advantage compared to shorter sugar-chains or single sugars, since by the use of starch the osmotic pressure of the medium in a bacterial culture is much lower, therefore the provided energy in a culture medium containing starch instead of smaller sugars is much higher at the same osmotical pressure.

A similar effect may be obtained by the usage of cellulose, glycogene, laminarine, leukosine, paramylone, inuline, pectine, and further long-chain sugars, as long as these sources can be made utizable for cultivated bacteria.

One possibility to make them utilizable is to provide the bacteria with enzymes enabling them to discompound the long sugar-chains step by step, resulting in smaller or single sugars, taken up and metabolized by the bacteria.

Since the discompounding of the long sugar-chains has to be carried out in the medium outside of the bacterium, the bacterium has to secret the according enzyme(s).

When the sugar-chain is unbranched, it may be sufficient to provide one enzyme for decomposition, which is specific for the bond between the single sugars of the chain. Such an enzyme is for example a glucosidase, specific for α1 → 4, β1 → 4, or β1 → 3 glucosidic bond. Such glucosidases decomposing starch are also named amylases.

Branched sugar-chains require at least one enzyme more, which can cleave the "branching" bond α1 → 6 in a sugar chain.

In some cases sugar-chains are first cut in smaller pieces, comprising for example 4 to 8 sugar molecules, by one enzyme, whereafter these "oligosugars" are decomposed by another enzyme resulting in single sugar molecules.

An example of such a "two-step decomposition" is the co-operation of α-amylase and β-amylase. α-amylase cleaves starch, resulting in "oligosugars" of 5 to 7 sugar-molecules whereby β-amylase decomposes starch as well as the resulting oligonucleotides in maltose, consisting of two bonded glucose-molecules. Maltose is cleaved by a further glucosidase, named maltase resulting in two molecules of glucose.

"Starch" as used herein comprises amylose as well as amylopectine.

Starch may be obtained by potatoes, corn, maize, cereal, rice, or any other source.

A preferred enzyme decomposing starch according to the present invention is amylase, more preferred amylase of Streptomyces griseus, most preferred α-amylase, encoded by amy gene of Streptomyces griseus.

Such a protein can be expressed for example by a bacterium containing the the encoding gene.
A bacterium naturally not containing such a gene can be transformed by introducing a heterologous gene encoding the desired protein.

Therefore, the heterologous gene is isolated from an organism, naturally expressing the desired protein, inserted in a suitable vector and introduced in the target organism, which is in the herein described invention Corynebacterium glutamicum.

DNA techniques like PCR, gene isolation, construction of a suitable plasmid, transformation and selection of a positive clone can be carried out according to any known method, the used techniques are not limiting the invention.

In the plasmid the desired gene may be under control of any promoter recognized by the host bacterium, however an inducible promoter is preferred.

An inducible promoter has the advantage that the bacterium can be adapted to medium conditions before the heterologous protein is expressed.

Any inducible promoter can be used accordingly the present invention, much of them are known in the art. However, a preferred inducible promoter is inducible by IPTG (isopropyl-β-D-thio-galactopyranoside). IPTG is added to external medium and taken up by the bacterium.

A further transformation of the bacterial strain of the present invention, for example transformation by any heterologous gene which protein product shall be produced in qualitatively and quantitatively high amounts, is practicable at any time. Further preferred products of such a strain containing at least one heterologous gene are peptides, amino acids and metabolites of biochemical pathways. Either products can be obtained naturally produced by the bacterium or products produced by the introduced heterologous gene(s).

### Legends to the figures:

### Figure 1:

### Plasmid maps of (a) pAmy and (b) pIAmy2

### Figure 2:

Amylase activity of Corynebacterium glutamicum strains harbouring different amy vectors. Quantification of the enzyme activity in culture supernatant of the Corynebacterium glutamicum strains RES167, Corynebacterium glutamicum RES167/pAmy, Corynebacterium glutamicum AMY2 (AMY2) grown in TYPS medium for 3 days. Determination of amylase activity is carried out by 30 min incubation of culture supernatant with a 2% solution of starch in 10 mM phosphate buffer (pH 7.0) at 37°C. Starch is thereby decomposed by amylase, resulting in an higher amount of reducing sugar molecules in solution. Proof of these reducing molecules is carried out by the method with dinitrosalicylic acid (Miller, 1959, Analytical chemistry 31: 426-428). Activity was determined 5 times, 1 mU was defined as 1 nmol reducing sugar min⁻¹ ml⁻¹.

The following examples shall only be considered as explaining the present invention in detail, not for restricting the scope of invention.

### Example 1:

### Construction of expression vectors pXT99A and pEC-XT99A

E. coli expression vector pTRC99A (Amann et al., 1988, Gene 69: 301-315) was cleaved with BspHI and treated with Klenow fragment. Tetracycline gene from C. glutamicum plasmid pAG1 (GeneBank Acc.No. AF121000) was inserted instead of ampicilline gene by ligation with T4 ligase, resulting in pXT99A. Ligation mix was electroporated into E. coli DH5αMCR.

For construction of pEC-XT99A E. coli - C. glutamicum shuttle vector the following cloning steps are carried out:

From plasmid pGA1 (Sonnen et al., 1991, Gene 107; 69-74) a 3484 bp fragment containing a replicon of C. glutamicum was obtained by restriction with BalI and PstI. This fragment was inserted into SmaI/PstI cleaved vector pK18mob2 (Tauch et al., 1998, Archives of microbiology 169: 303-312). After religation a 839 bp fragment of the inserted replicon fragment was deleted by cleavage with BamHI/XhoI and the vector fragment was treated with Klenow fragment. After religation of the vector a 2645 bp KpnI/PstI, Klenow treated fragment, containing C. glutamicum minimal replicon was inserted into plasmid pXT99A, cleaved with NheI and treated with Klenow-polymerase. Ligation was carried out as described above. After electroporation into C. glutamicum plasmid pEC-XT99A was isolated and verfied.

### Example 2:

### Construction of amylase producing Corynebacterium glutamicum strains

To construct an amylase secreting Corynebacterium glutamicum strain, the vector pULMJ95 (Cadenas, R.F., Fernandez-Gonzales, C., Martin, J.F. and Gil, J.A. (1996), FEMS Microbiol. Lett. 137, 63-68) was digested with EcoRI and Ecl136II and a 2,1 kb fragment harbouring the promoterless amy gene of Streptomyces griseus IMRU 3570 was cloned into the EcoRI and Ecl136II cleaved E. coli / Corynebacterium glutamicum shuttle expression vector pEC-XT99A under control of the IPTG-inducible trc promoter (Amann, E., Ochs, B. and Abel, K.-J. (1988), Gene 69: 301-315). The new constructed vector pAmy with IPTG inducible amylase expression was electroporated to Corynebacterium glutamicum (ATCC 13032).

For Corynebacterium glutamicum strains harbouring a chromosomal copy of the amylase gene, promoterless amy was cloned as described above into the E. coli expression vector pXT99A. In a second step the XbaI and HindIII gene fragment of dciAE from pLW60 (Wehmeier, L., Schäfer, A., Burkowski, A., Krämer, R., Mechold, U., Malke, H., Pühler, A. and Kalinowski, J. (1998), Microbiology 144, 1853-1862) was cloned into the resulting vectors downstream of amy. The new non-replicative plasmid pIAmy2 was integrated into the Corynebacterium glutamicum chromosome by electroporation and following homologous recombination, resulting in the strain Corynebacterium glutamicum AMY2 (Table 1).

**Table 1 :**

| Corynebacterium glutamicum strains and plasmids: | | |
|---|---|---|
| **strains/plasmids** | **relevant genotype** | **reference** |
| **strains:** | | |
| ***C. g.* RES167** | **Restriction-deficient mutant of ATCC 13032, *Δ*(*cglRI- cglRII*)** | **Universität Bielefeld** |
| ***C. g.* AMY2** | **RES167 *dciAE* :: pIAmy2, Tc**^{**R**}**, test strain for amylase excretion** | **This study** |

| **plasmids:** | | |
|---|---|---|
| **pLW60** | **pK18*mob* harbouring *dciAE* fragment** | **Wehmeier *et al*., (1998)** |
| **pXT99A** | ***E. coli* Expression vector, P**_{**trc**}**, Tc**^{**R**} | **Universität Bielefeld** |
| **pEC-XT99A** | ***E. coli* / *C. glutamicum* shuttle expression vector, P**_{**trc,**} **Tc**^{**R**} | **Universität Bielefeld** |
| **pULMJ95** | ***E. coli* / *C. glutamicum* shuttle vector, *amy*, Km**^{**R**} | **Cadenas *et al.* (1996)** |
| **pAmy** | **pEC-XT99A harbouring *amy* downstream P**_{**trc**} | **This study** |
| **pIAmy2** | **pXT99A harbouring *amy dciAE*-fragment downstream P**_{**trc**} | **This study** |

### Example 3:

### Amylase secretion of Corynebacterium glutamicum RES167 and Corynebacterium glutamicum pAmy and Corynebacterium glutamicum AMY2

For performing amylase assay Corynebacterium glutamicum strains were cultivated in solid and liquid cultures of TYPS medium, consisting of 1 % tryptone (Difco) 1 % yeast extract (Difco), 1 % peptone (Difco) and 2 % soluble starch (Sigma). Amylase production was induced by adding 50nM IPTG to the TYPS medium. For measurement of intracellular amylase activity, Corynebacterium glutamicum grown in liquid culture were washed twice in 10mM phosphate buffer (pH 7,0). To disrupt the cells a Ribolyser (Hybaid) was used two times for 30 s at a speed of 6.

Amylase activity was measured by a modification of the dinitrosalicylic method (Miller, G.L., 1959, Anal. Chem. 31, 426-428) in the supernatant of Corynebacterium glutamicum strains. The assay was carried out at 37°C for 30 min with 2 % soluble starch in 10mM phosphate buffer (pH 7,0). The volume activity (mU) was defined as nmol reducing sugar min⁻¹ ml⁻¹. Starch degradation was assayed on agar plates by colouring with Lugols solution. Amylase activity was detected as clearing zones around the colonies.

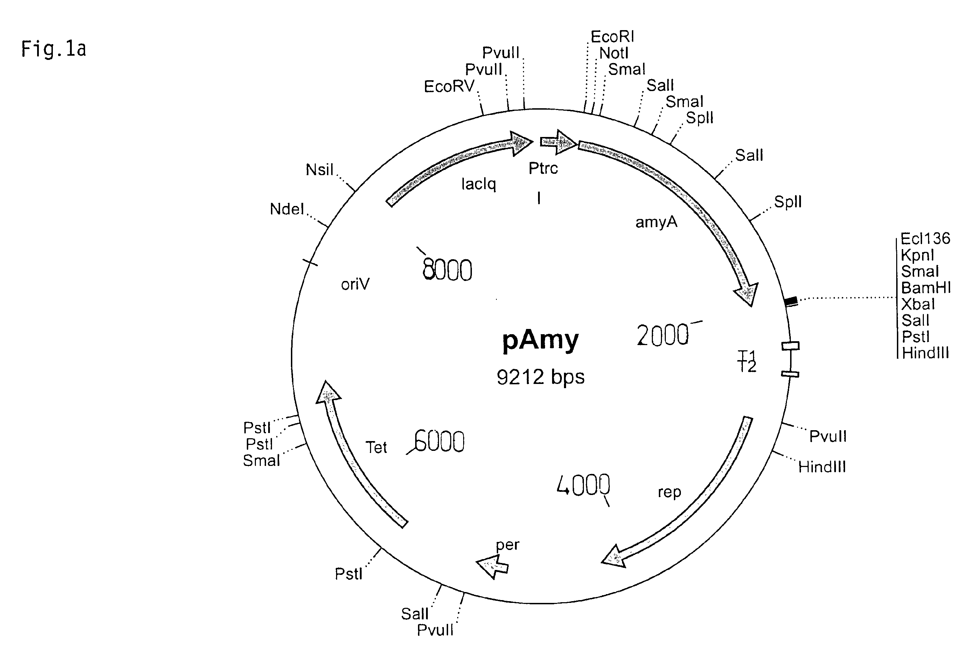

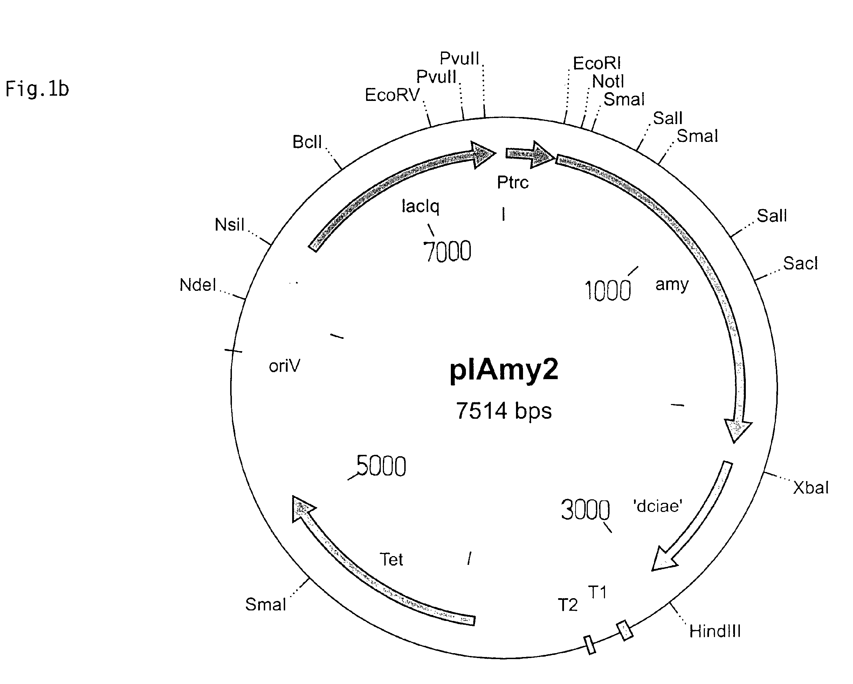

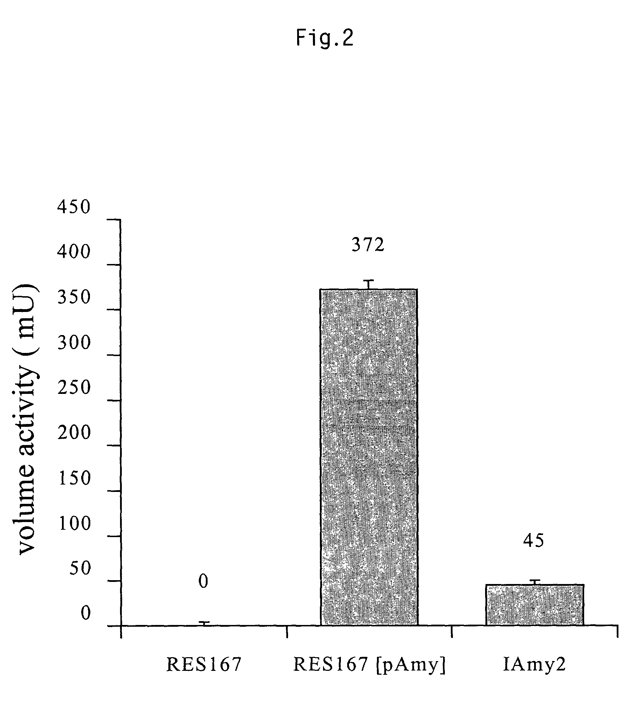

Amylase activity was determined on starch containing agar plates (data not shown). To quantify the enzyme activity, the supernatant of Corynebacterium glutamicum RES167/pAmy grown in liquid culture was tested. After three days of growth a maximal volume activity of 372 mU could be measured (Fig. 2). No amylase activity could be detected in the wild type, neither on agar plates nor in culture supernatant (Fig. 2) leading to the conclusion that Corynebacterium glutamicum RES167 is not able to degrade starch.

Starch degradation on agar plates was detectable for Corynebacterium glutamicum AMY2 and amylase activity in the culture supernatant after three days of incubation was 45 mU (Fig. 2). Corynebacterium glutamicum AMY2, bearing a chromosomal copy of amy, secrets only 12 % of the amylase produced by Corynebacterium glutamicum RES167/pAmy. This led to the conclusion, that the plasmid pAmy has around 8 copies per cell in Corynebacterium glutamicum. In opposite to the wild type strain, all amylase producing Corynebacterium glutamicum strains are able to grow on minimal media with starch as only carbon source. It was concluded that amylase production is easy in both, a replicative and an integrated system in Corynebacterium glutamicum.

## Claims

1. Genetically modified Corynebacterium glutamicum strain, **characterized in that** the strain is able to use starch as sole carbon source.

2. Bacterial strain of claim 1, **characterized in that** the strain contains a heterologous amylase gene.

3. Bacterial strain of claim 2, whereby the amylase gene is amy gene of Streptomyces grisues.

4. Bacterial strain according to any of claims 2 or 3, whereby the amylase gene is either integrated in the chromosome (cis-orientation) or contained on a plasmid (trans-orientation) in the bacterium.

5. Bacterial strain according to any of claims 2 to 4, wherein the amylase gene expression is inducible.

6. Bacterial strain of claim 5, whereby gene expression is inducible by IPTG.

7. Bacterial strain according to any of claims 1 to 6, containing at least one further heterologous gene.

8. Use of a bacterial strain of one of claims 1 to 7 for the production of a desired product selected from proteins, peptides, amino acids and metabolites of biochemical pathways.
